(19) Europäisches Patentamt
European Patent Office
Office européen des brevets

(11) EP 3 305 956 A1

(12) EUROPEAN PATENT APPLICATION
published in accordance with Art. 153(4) EPC

(43) Date of publication:
11.04.2018 Bulletin 2018/15

(51) Int Cl.:
*D04B 1/16* (2006.01)       *D03D 15/00* (2006.01)
*D04B 1/28* (2006.01)       *A41D 19/00* (2006.01)
*A41D 19/015* (2006.01)

(21) Application number: 16800088.3

(22) Date of filing: 26.05.2016

(86) International application number:
PCT/JP2016/065564

(87) International publication number:
WO 2016/190384 (01.12.2016 Gazette 2016/48)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 27.05.2015 JP 2015107214

(71) Applicant: Toray Industries, Inc.
Tokyo 103-8666 (JP)

(72) Inventors:
• SAKAI, Kenichi
  Otsu-shi
  Shiga 520-2141 (JP)
• MATSUMURA, Kazuya
  Tokyo 103-8666 (JP)
• KAJIYAMA, Hiroshi
  Otsu-shi
  Shiga 520-2141 (JP)

(74) Representative: Kador & Partner
Corneliusstraße 15
80469 München (DE)

(54) **FABRIC**

(57) The present invention aims to provide a fabric that can improve the gripping performance of gripping of a contacted object via a glove when the fabric is made into the glove, and that can facilitate wearing and taking off the glove.

The present invention is a fabric including a filament yarn A having a single fiber diameter of 100 to 1000 nm and a filament yarn B having a single fiber diameter of 10 $\mu$m or more, wherein a value obtained by dividing an exposed area of the filament yarn A on a first surface of the fabric by an exposed area, on the first surface, of all fibers exposed on the first surface is 0.50 or more and 0.90 or less.

**Description**

TECHNICAL FIELD

The present invention relates to a fabric, and particularly to a fabric suitable for gloves.

BACKGROUND ART

In recent years, fabrics using nanofibers having a single fiber diameter of less than 1 micrometer have been proposed pursuing delicate touch and soft feel. Since nanofibers have a single fiber diameter of the order of nanometers, the surface area of a fabric using nanofibers is dramatically larger than the surface area of a fabric using fibers having a large single fiber diameter. As a result, it is possible to develop a high static friction coefficient which could not be obtained with a fabric using fibers having a large single fiber diameter. Patent Document 1 discloses a woven or knitted fabric containing nanofibers and exhibiting a high surface static friction coefficient.

PRIOR ART DOCUMENT

PATENT DOCUMENT

Patent Document 1: Japanese Patent Laid-open Publication No. 2009-24278

SUMMARY OF THE INVENTION

PROBLEM TO BE SOLVED BY THE INVENTION

In the woven or knitted fabric disclosed in Patent Document 1, in order to enhance the firmness of contact between the woven or knitted fabric and a contacted object with which the surface is in contact, a lot of nanofibers are exposed on a contact surface at which the woven or knitted fabric is in contact with the contacted object. Hereinafter, an object with which the surface of the fabric is in contact will be referred to as a contacted object.

Further, Patent Document 1 only discloses a woven or knitted fabric in which only nanofibers are exposed on both surfaces (hereinafter referred to as "fabric I"), and a woven or knitted fabric in which only nanofibers are exposed on one surface and only fibers other than the nanofibers are exposed on the other surface (hereinafter referred to as "fabric II") . Here, in the case of the fabric I, a value obtained by dividing the exposed area, on a first surface of the fabric, of nanofibers exposed on the first surface by an exposed area, on the first surface of the fabric, of all fibers exposed on the first surface (hereinafter referred to as "exposure ratio of nanofibers on the first surface") is 1, and a value obtained by dividing the exposed area, on a second surface of the fabric, of nanofibers exposed on the second surface by an exposed area, on the second surface of the fabric, of all fibers exposed on the second surface (hereinafter referred to as "exposure ratio of nanofibers on the first surface") is 1. In the case of the fabric II, the exposure ratio of nanofibers on the first surface is 1, and the exposure ratio of the nanofibers on the second surface is 0. That is, since the nanofibers are excessively exposed on at least one surface of the fabric described in Patent Document 1, the static friction coefficient of the surface of the fabric on which the nanofibers are excessively exposed becomes excessively large. As a result, in the manufacturing process of this fabric, there is a problem that wrinkles and glazing are generated in the fabric and the surface quality of the fabric deteriorates.

In view of the above problems, the present invention aims to provide a fabric that can improve the gripping performance of gripping of a contacted object via a product (for example, a glove) that is to be interposed between fingers and the contacted object when the fabric is made into the glove, and has an excellent surface quality.

SOLUTIONS TO THE PROBLEM

In order to solve the above problems, the present invention provides the following fabrics.

(1) A fabric including a filament yarn A having a single fiber diameter of 100 to 1000 nm and a filament yarn B having a single fiber diameter of 10 $\mu$m or more, wherein a value obtained by dividing an exposed area of the filament yarn A on a first surface of the fabric by an exposed area, on the first surface, of all fibers exposed on the first surface is 0.50 or more and 0.90 or less.
Further, there are the following preferred embodiments.
(2) The fabric described in (1), wherein a value obtained by dividing an exposed area of the filament yarn A on a second surface of the fabric by the exposed area, on the second surface, of all fibers exposed on the second surface is 0.10 or more and 0.40 or less.

(3) The fabric described in (2), wherein a value obtained by dividing a static friction coefficient of the first surface in a wet state by a static friction coefficient of the second surface in a wet state is 1.2 or more and 2.5 or less, and the static friction coefficient of the first surface in the wet state is 0.7 or more.

(4) The fabric described in any one of (1) to (3), wherein a tensile stress at 30% elongation in a wet state is 100N/50 mm or less.

(5) The fabric described in any one of (1) to (4), wherein the filament yarn A is a polyamide fiber and has a single fiber diameter of is 100 to 300 nm.

(6) A glove including the fabric described in any one of (1) to (5), wherein the fabric has a thickness of 0.2 to 0.9 mm.

(7) The glove described in (6) used for surgery.

EFFECTS OF THE INVENTION

According to the present invention, it is possible to provide a fabric that can improve the gripping performance of gripping of a contacted object via a glove when the fabric is made into the glove, and that can facilitate wearing and taking off the glove.

EMBODIMENTS OF THE INVENTION

Hereinafter, embodiments of the present invention will be described in detail.

The fabric of the present invention is constituted by a filament yarn A having a single fiber diameter of 100 to 1000 nm and a filament yarn B having a single fiber diameter of 10 $\mu$m or more, and a value obtained by dividing an exposed area of the filament yarn A on a first surface of the fabric by an exposed area, on the first surface, of all fibers exposed on the first surface is 0.50 or more and 0.90 or less.

The fabric of the present invention may consist of only the filament yarn A and the filament yarn B described above or may include a material other than the filament yarn A and the filament yarn B as long as the effect of the present invention is not degraded.

The filament yarn A used in the present invention will be described. The single fiber diameter of the filament yarn A is 100 to 1000 nm. When the filament yarn A has a single fiber diameter of 100 nm or more, deterioration in abrasion resistance caused by decrease in fiber strength of the filament yarn A can be suppressed. In addition, by setting the single fiber diameter of the filament yarn A to 1000 nm or less, it is possible to increase the static friction coefficient on a contact surface of the fabric with a contacted object. Further, by setting the single fiber diameter of the filament yarn A to 500 nm or less, the difference between the static friction coefficient of the fabric in a dry state and the static friction coefficient of the fabric in a state wet with water can be made small. In general fabrics, the static friction coefficient between the fabric in the wet state and the contacted object tends to be lower than the static friction coefficient of the fabric in the dry state. However, in the fabric of the present invention, the static friction coefficient between the fabric in the wet state and the contacted object is close to the static friction coefficient in the fabric in the dry state. Although the reason for this is not clear, when the fiber diameter of the fibers constituting the fabric is small, voids generated between the fibers also become small. It is presumed that this is because, if the voids present in the fabric are small, force of capillary phenomenon by which the fabric absorbs water increases, and force by which the fabric itself is attracted to the contacted object increases as a reaction to this. From the above viewpoint, the upper value of the single fiber diameter of the filament yarn A is more preferably 500 nm or less. The upper value of the single fiber diameter of the filament yarn A is further more preferably 300 nm or less. From the above viewpoint, the lower value of the single fiber diameter of the filament yarn A is more preferably 200 nm or more.

As a method for obtaining the filament yarn A used in the fabric of the present invention, for example, a method of manufacturing a composite fiber using a distribution-plate-type composite spinneret as follows can be employed. As such a composite fiber, a sea-island type composite fiber produced by the method of manufacturing a composite fiber disclosed in WO 2012/173116 is preferably used.

The type of the polymer forming the filament yarn A used in the fabric of the present invention is not particularly limited, but it is preferably a thermoplastic polymer. The above-mentioned polymer may be a copolymer of two or more kinds of monomers, or two or more kinds of polymers may be friends. In addition, the polymer may also contain an additive such as a stabilizer. In order to improve availability, polyamide such as nylon 6 or polyethylene terephthalate is more preferable as the above-mentioned polymer. In the case where a step of obtaining the filament yarn A includes a step of removing an easily soluble sea component with an alkaline solution, it is more preferable that the above-mentioned polymer is polyamide because polyamide shows poor solubility in an alkaline solution and can be handled easily. In this case, the filament yarn A is a polyamide fiber. Furthermore, by using polyamide as the above-mentioned polymer, the static friction coefficient of the contact surface of the fabric with the contacted object becomes higher than in the case where a hydrophobic fiber such as polyethylene terephthalate is used as the filament yarn A even in the case where the fabric is in a state wet with a lipid-containing liquid.

Although the fiber form of the filament yarn A used for the fabric of the present invention is not particularly limited, the filament yarn A is preferably a multifilament yarn of long fiber. In the case where the filament yarn A is a multifilament yarn, the cross-sectional shape of a single yarn constituting the filament yarn A is not particularly limited, and may be a cross-sectional shape such as a circle, a triangle, a hexagon, a Y shape, a flattened shape, a hollow shape, or the like.

When the filament yarn A is a multifilament yarn, although the number of filaments is not particularly limited, but the number is preferably 500 or more in order to obtain the texture of the fabric derived from the nanofibers. Here, the number of filaments of the filament yarn A also includes the number of filaments doubled when making the fabric. More preferably, the lower value of the number is 10,000 or more, and the upper value of the number is 100,000 or less.

Further, in the case where the filament yarn A is a multifilament yarn, the total fineness of the filament yarn A is preferably 10 to 400 dtex. By setting the total fineness to 10 dtex or more, the mechanical strength of the fabric such as tensile strength and tear strength can be made to be more excellent, and breakage of the fabric at the time of sewing or molding the fabric can be suppressed. In addition, by setting the total fineness of the filament yarn A to 400 dtex or less, it is possible to suppress the decrease in elongation of the fabric and to improve the setting property of the fabric.

Further, in the case where sea-removal treatment is assumed, the filament yarn A is obtained by subjecting a sea-island composite fiber containing the filament yarn A as an island component to alkali weight reduction treatment. And the sea component of the above-mentioned sea-island composite fiber can be selected from polymers which are melt-moldable and exhibit higher solubility in liquid than other components. As the sea component, copolymerized polyester, polylactic acid, polyvinyl alcohol and the like which exhibit high solubility in an aqueous solvent or hot water are preferable. In particular, it is preferable to use polyester in which either one or both of polyethylene glycol and sodium sulfoisophthalate are copolymerized or polylactic acid because the polyester or polylactic acid can be easily dissolved in an aqueous solvent that has spinnability and low concentration. Also, from the viewpoint of sea removability and spreadability of generated microfibers, polyester in which sodium sulfoisophthalate is independently copolymerized is particularly preferable. In the case where a copolyester obtained by copolymerization of sodium sulfoisophthalate or the like, a polylactic acid or the like is used as the sea component, an aqueous alkaline solution such as an aqueous solution of sodium hydroxide can be used.

Next, the filament yarn B used in the present invention will be described. The filament yarn B has a single fiber diameter of 10 $\mu$m or more. By setting the single fiber diameter of the filament yarn B to 10 $\mu$m or more, the form stability and texture can be improved. From the above viewpoint, the single fiber diameter of the filament yarn B is preferably 15 $\mu$m or more. In addition, although the upper value of the single fiber diameter of the filament yarn B is not particularly limited, the upper value is preferably 20 $\mu$m or less. By setting the single fiber diameter of the filament yarn B to 20 $\mu$m or less, the flexibility of the fabric can be further improved.

As a method for obtaining the filament yarn B, a melt spinning method can be employed. Specifically, a polymer melted by heating at a temperature equal to or higher than the melting point of the polymer in a heating cylinder is extruded from a spinneret, the extruded yarn is cooled, stretched, then cut or wound as it is, and thus the filament yarn B can be obtained.

Although the type of polymer forming the filament yarn B is not particularly limited, but the polymer is preferably a thermoplastic polymer. The above-mentioned polymer may be copolymerized with another component. In addition, the polymer may also contain an additive such as a stabilizer. Polyamide and polyester are preferred for improvement of availability and handling during production. More preferably, the polymer is nylon 6 or polyethylene terephthalate.

Although the fiber form of the filament yarn B is not particularly limited, the filament yarn B is preferably a long fiber (multifilament yarn) . When the filament yarn B is a long fiber, the filament yarn B may be false-twisted or covered with polyurethane elastic fiber. As a method of false twisting, any method such as pin twisting, friction twisting, nip belt twisting, air twisting or the like may be used. The heating heater may be either a contact type or a non-contact type. The cross-sectional shape of the filament yarn B is not particularly limited, and may be a cross-sectional shape such as a circle, a triangle, a hexagon, a Y shape, a flattened shape, a hollow shape, or the like.

In the case where the filament yarn B is a multifilament yarn, the number of filaments is not particularly limited, but the number is preferably 5 or more in order to achieve an appropriate rigidity for the fabric. From the above viewpoint, more preferably, the lower value is 10 or more, and the upper value is 70 or less.

In the case where the filament yarn B is a multifilament yarn, the total fineness (the product of the single fiber fineness and the number of filaments) of the filament yarn B is preferably in the range of 10 to 200 dtex. By setting the total fineness to 10 dtex or more, the mechanical strength of the fabric such as tensile strength and tear strength can be made to be more excellent, and breakage of the fabric at the time of sewing or molding the fabric can be suppressed. Also, by setting the total fineness of filament yarn B to 200 dtex or less, it is possible to suppress the decrease in elongation of the fabric and to improve the setting property of the fabric.

Next, the fabric of the present invention will be described.

As a form of the fabric of the present invention, a woven fabric, a knitted fabric or a nonwoven fabric can be employed. The type of the woven fabric can be any of three basic weaves such as plain weave, twill weave and satin weave, and modified weaves, entwined weaves, pile weaves, and textile weaves based on the three basic weaves, and these can be also employed in combination. The type of the knitted fabric may be a warp knitted fabric or a weft knitted fabric. Examples of the texture of the warp knitted fabric include tricot knitting, Raschel knitting, jacquard knitting, and the like, examples of the texture of the weft knitted fabric include plain stitch, rib stitch, interlock stitch, pearl stitch, tuck stitch and the like, and these can be also combined. Examples of the combination include circular interlock knitting, moss stitch, and the like. It should be noted that the knitting can be performed by a usual method using a conventional knitting machine such as a circular knitting machine, a flat knitting machine, a tricot knitting machine, a Raschel knitting machine, or the like. The number of layers is also not particularly limited and the fabric may be a knitted fabric having a single layer or a multilayer structure of two or more layers . Examples of the nonwoven fabric include a needle punching method and a spunlacing method. Since the fitting property to the contacted object can be improved, the fabric is preferably a knitted fabric. Since productivity can be improved, the fabric is more preferably a circular knitted fabric.

In the case where the fabric of the present invention is a knitted fabric, the filament yarn A and the filament yarn B may be interlaced, and in the case where the fabric is a woven fabric, the filament yarn A and the filament yarn B may be interwoven. In the case where the fabric is a nonwoven fabric, the filament yarn A and the filament yarn B may be mixed. Further, a knitted fabric or a woven fabric may be constituted by a mixed yarn composed of the filament yarn A and the filament yarn B.

In the case where the filament yarn A contained in the fabric of the present invention is derived from a composite fiber containing a slightly soluble island component and an easily soluble sea component, a method for making the composite fiber into nanofibers is not particularly limited, and the composite fiber may be made into a fabric form and then immersed in an aqueous alkaline solution as sea removal treatment. At this time, the alkaline aqueous solution is preferably heated to 50°C or higher because this accelerates the progress of hydrolysis of the easily soluble sea component. From the above viewpoint, more preferably, the temperature of the alkaline aqueous solution is 80°C or higher. In the case of using an aqueous sodium hydroxide solution as the aqueous alkaline solution, the concentration of sodium hydroxide is preferably 0.5 to 5%. In addition, it is preferable to perform treatment by using, for example, a jet dyeing machine, for industrial reasons because this enables processing a large amount at one time and freely setting the bath ratio and thus improves the productivity. In the case where the sea removal treatment is carried out using the jet dyeing machine described above, it is preferable that the fabric (hereinafter referred to as "fabric precursor") before the sea removal treatment has a tubular form such as a circular knitted fabric. In this case, it is preferable that the fabric precursor is set in the jet dyeing machine such that a surface to be the first surface of the fabric of the fabric precursor is set to the inside of the tubular form, and a surface to be the second surface of the fabric of the fabric precursor is set to the outside of the tubular form. In this case, the surface to be the second surface of the fabric of the fabric precursor comes into contact with a tube of the dyeing machine. By setting the fabric precursor in the jet dyeing machine as described above, it is possible to suppress adsorption of the first surface having many filament yarns A to the tube of the dyeing machine tube caused by the high static friction coefficient thereof after the sea removal treatment, and thus the process passability is further improved.

The fabric of the present invention may be subjected to a dyeing process before, after, or before and after the sea removal treatment with the alkaline aqueous solution. Calendar processing or embossing may be applied, and further napping processing or water-repelling processing may be performed. Furthermore, functions of such as deodorant, antistatic agent, antibacterial agent and the like may be imparted by post-processing. In particular, the napping processing can improve the static friction coefficient of the fabric in a dry state. A method for napping is not particularly limited, and examples thereof include card cloth raising, and emery raising using sandpaper.

When the polymer of the filament yarn A and the filament yarn B is nylon 6, the temperature of the fabric of the present invention at the time of finishing setting is preferably 150°C or less. When the temperature exceeds 150°C, process passability is lowered due to heat sag of the fabric. From the above viewpoint, the temperature is more preferably 130°C or less. To be noted, the lower value is 100°C or higher.

In the fabric of the present invention, a value obtained by dividing the exposed area of the filament yarn A on the first surface by the exposed area, on the first surface, of all fibers exposed on the first surface (hereinafter referred to as a first value) is 0.50 or more and 0.90 or less. Here, by setting the first value to 0.50 or more, the static friction coefficient can be improved. From the above viewpoint, the first value is preferably 0.60 or more. Meanwhile, by setting the first value to 0.90 or less, deterioration of the surface quality of the first surface of the fabric caused by occurrence of wrinkles and glazing during a manufacturing process can be suppressed. From the above viewpoint, the first value is preferably 0.80 or less. In the case of making the fabric of the present invention into a glove, a glove excellent in surface quality can be obtained by making the glove such that the first surface of the fabric is on the outside (object gripping side) of the glove.

In addition, in the fabric of the present invention, a value obtained by dividing the exposed area of the filament yarn A on the second surface by the exposed area, on the second surface, of all fibers exposed on the second surface (hereinafter referred to as a second value) is preferably 0.10 or more and 0.40 or less. Here, by setting the second value to 0.10 or more, the static friction coefficient can be improved. More preferably, from the above viewpoint, the second value is 0.20 or more. Meanwhile, by setting the second value to 0.40 or less, it is possible to suppress the decrease in process passability of the fabric and facilitate wearing and taking off a glove in the case where the fabric is made into the glove. More preferably, from the above viewpoint, the second value is 0.30 or less.

Here, the embodiment of the fabric of the present invention in which the first value is 0.50 or more and 0.90 or less and the second value is 0.10 or more and 0.40 or less has a large static friction coefficient on the first surface, and there is an appropriate difference between the static friction coefficient of the first surface of the fabric and the static friction coefficient of the second surface of the fabric. Such a fabric is a fabric which can be suitably used for a product that is required to have a large static friction coefficient on one surface and an appropriate difference in static friction coefficient between the one surface and the other surface. A glove can serve as an example of the product that is required to have a large static friction coefficient on one surface and an appropriate difference in static friction coefficient between the one surface and the other surface of a glove as described above. Depending on the application, the contacted object grasped by a wearer of the glove is apt to fall due to slippage occurring between the outer surface of the glove and the contact surface of the contacted object with the glove. In order to suppress this, a large static friction coefficient is required on the outer surface of the glove. Meanwhile, with regard to the inner surface of the glove, the glove becomes difficult to wear and take off in the case where the inner surface of the glove has a static friction coefficient similar to the outer surface of the glove. Therefore, the static friction coefficient of the inner surface of the glove needs to be somewhat lower than the static friction coefficient of the outer side of the glove. Meanwhile, in the case where the static friction coefficient of the inner surface of the glove is too small, slippage occurs between the hand of the wearer of the glove and the inner surface of the glove, and it becomes difficult to grip the contacted object via the glove. Therefore, it is necessary that the static friction coefficient of the inner surface of the glove is also large to some extent. That is, as described above, it is necessary for the glove to have a large static friction coefficient on the outer surface of the glove and an appropriate difference between the static friction coefficient of the outer surface of the glove and the static friction coefficient of the inner surface of the glove.

In addition, in the case where the static friction coefficient of both of the first surface and the second surface of the fabric is large, the fabric in a wet state is adsorbed onto a guide roller for feeding or in pot pipe used in alkali reduction in the manufacturing process thereof, and thus manufacturing the fabric becomes difficult. Therefore, the fabric of the present invention is configured to have a large static friction coefficient on the first surface of the fabric and an appropriate difference between the static friction coefficient of the first surface of the fabric and the static friction coefficient of the second surface of the fabric, and the second surface of the fabric is configured to be in contact with the guide roller and the inside of the pot pipe in the manufacturing process. Thus, the adsorption of the fabric onto the guide roller and the inside of the pot pipe can be suppressed, and decrease in the process passability can be suppressed.

Further, one embodiment of the fabric of the present invention, wherein the first value is 0.50 or more and 0.90 or less and the second value is 0.10 or more and 0.40 or less, solves the problem of the fabric described in Patent Document 1. Here, problems of the fabric described in Patent Document 1 are as follows. As described above, the fabric I and fabric II are described in Patent Document 1. In the fabric I, the exposure ratio of nanofibers on the first surface is 1.00 and the exposure ratio of the nanofibers on the second surface is 1.00. Further, in the fabric II, the exposure ratio of nanofibers on the first surface is 1.00 and the exposure ratio of the nanofibers on the second surface is 0.

Here, for example, when the fabric I is processed into a glove, the first surface or the second surface is disposed as a surface that comes into contact with a hand. Further, in the fabric I, both the exposed ratio of nanofibers on the first surface and the exposed ratio of the nanofibers on the second surface are 1.00. Therefore, when wearing and taking off the glove, there is a problem that the static friction coefficient between the hand and the glove is large and it is difficult to wear or take off the glove.

Further, in the fabric I, only the nanofibers are exposed on both surfaces thereof. As a result of this, the static friction coefficient of both surfaces becomes large, and there is a problem that the fabric I in a wet state in the manufacturing process is adsorbed on the guide roller for feeding or in the pot pipe alkali reduction, which makes processing difficult.

Further, for example, when the fabric II is processed into a glove and the second surface of the fabric II is disposed as a surface in contact with the hand, only the nanofibers are exposed on the surface of the glove contacting the contacted object. Since the static friction coefficient between the contacted object and the glove is large when using the glove to grasp the contacted object, sliding down of the grasped contacted object is suppressed. On the other hand, since the static friction coefficient between the hand and the glove becomes small, there is a problem that slippage occurs between the hand and the glove, and it is difficult to grasp the contacted object via the glove. The fabric of the present invention preferably has a basis weight of 50 to 350g/m$^2$. By setting the basis weight of the fabric to 50g/m$^2$ or more, the form stability of the fabric improves and handling property of the fabric at the time of use improves. Further, by setting the basis weight of the fabric to 350g/m$^2$ or less, elongation of the fabric is improved and generation of wrinkles at the time of heat setting in the manufacturing process can be suppressed. The lower value is more preferably 60g/m$^2$ or more, and still more preferably 70g/m$^2$ or more. The upper value is more preferably 300g/m$^2$ or less, and still more preferably 250g/m$^2$ or less.

The fabric of the present invention preferably has a thickness in the range of 0.2 to 3.0 mm. By setting the thickness of the fabric within the range described above, the static friction coefficient of the contact surface of the fabric with the contacted object can be increased, and the difference between the static friction coefficient of the fabric in a dry state and the static friction coefficient of the fabric in a wet state can be made smaller. Although the reason for this is not clear, it is thought that this is because when the thickness of the fabric falls within the above range, shear deformation occurs in the fabric at the time of friction between the fabric and the contacted object, and the above static friction coefficient increases. Incidentally, by setting the thickness of the fabric to 0.2 mm or more, it is possible to suppress reduction the strength of the fabric and breakage occurring during the manufacturing process. Meanwhile, by setting the thickness of the fabric to 3.0 mm or less, when pressure is applied to the fabric from the other surface of the fabric in a state where the contacted object is in contact with one surface of the fabric, pressure is more easily transmitted to the contacted object from the other surface of the fabric, and the static friction coefficient of the contact surface of the fabric with the contacted object becomes larger. When a fabric having a thickness of 3.0 mm or less is used for touching the contacted object via the fabric, for example, as a glove, a wearer of the glove or the like can easily feel a sense of touch of the contacted object. From the above viewpoint, the lower value of the thickness of the fabric is more preferably 0.3 mm or more, and the upper value of the thickness is more preferably 0.9 mm or less. Furthermore preferably, the upper value is 0.7 mm or less.

The fabric of the present invention is preferably grade 3 or higher in terms of pilling property evaluated by a method prescribed in JIS L 1076 (2010). By using the filament yarn A and by adjusting the single fiber diameter thereof, the abrasion resistance of the fabric is improved and the pilling property can be grade 3 or higher. The pilling property of the fabric is more preferably grade 4 or higher.

In the fabric of the present invention, it is preferable that a value obtained by dividing a static friction coefficient of the first surface in a wet state by a static friction coefficient of the second surface in a wet state is 1.2 or more and 2.5 or less, and the static friction coefficient of the first surface in the wet state is 0.7 or more. Here, the static friction coefficient in the present application is a static friction coefficient measured on the basis of a method prescribed in in JIS P 8147 (2010) (8. inclination method) as will be described in examples, by setting a silicone plate (simulated skin) as the contacted object, and with a load of $29g/cm^2$. The static friction coefficient is the ratio of the weight of an object at the moment when the object starts to slide to the applied force, and as the numerical value thereof increases, the object becomes less slippery. By using the filament yarn A for the fabric and adjusting the single fiber diameter of the filament yarn A, the ratio of the exposed area of the filament yarn A on the first surface, the ratio of the exposed area of the filament yarn A on the second surface, the thickness of the fabric, and the like in an appropriate range, a fabric satisfying the ratio of the static friction coefficient and the range of the above values can be obtained. Further, the fabric of the present invention is preferably isotropic in terms of static friction coefficient.

By setting the value obtained by dividing the static friction coefficient of the first surface in the wet state by the static friction coefficient of the second surface in the wet state to 1.2 or more, a fabric in which the static friction coefficient of the first surface in the wet state is large and there is an appropriate difference between static friction coefficients of the first surface and the second surface of the fabric in the wet state can be obtained. From the above viewpoint, the lower value is more preferably 1.3 or more. The lower value is further more preferably 1.5 or more. Meanwhile, by setting the value obtained by performing division with the static friction coefficient of the second surface in the wet state to 2.5 or less, in the case where fabric is made into a glove and the glove is in the wet state, occurrence of slippage between the hand of the wearer of the glove and the inner surface can be suppressed and difficulty in gripping the contacted object via the glove can be suppressed. From the above viewpoint, the upper value is more preferably 2.3 or less.

Further, by setting the static friction coefficient of the first surface in the wet state to 0.7 or more, it is possible to obtain a fabric having high sliding resistance on the first surface in the wet state. From the above viewpoint, the lower value is more preferably 1.0 or more. In addition, although the upper value is not particularly limited, the upper value is preferably 2.5 or less because when the static friction coefficient is too large, conversely the contacted object such as the skin may be damaged. The upper value is more preferably 2.0 or less.

In addition, it is preferable that the fabric of the present invention has a static friction coefficient of 0.8 or more on the first surface thereof in a dry state of the fabric, and a value obtained by dividing the static friction coefficient of the first surface of the fabric in a wet state by the static friction coefficient of the first surface of the fabric in a dry state is 0.9 or more. In the present invention, by using the filament yarn A for the fabric and by adjusting the single fiber diameter of the filament yarn A, the exposure ratio of the filament yarn A on the first surface, the thickness of the fabric, and the like in appropriate ranges, a fabric satisfying the range of the static friction coefficient described above can be obtained.

By setting the static friction coefficient of the first surface of the fabric in a dry state to 0.8 or more, it is possible to obtain a fabric having high sliding resistance on the first surface thereof. The lower value is more preferably 0.9 or more. In addition, although the upper value is not particularly limited, the upper value is preferably 2.5 or less because when the static friction coefficient is too large, conversely the contacted object such as the skin may be damaged. The upper value is more preferably 2.0 or less.

Further, by setting a value obtained by dividing the static friction coefficient of the fabric in a wet state by the static friction coefficient of the fabric in a dry state to 0.9 or more, when a wearer grasps the contacted object via the fabric, the wearer can grasp the contacted object with the same force and sense in both cases where the fabric is in a dry state and where the fabric is in a wet state, and a fabric having high sliding resistance even in the wet state can be obtained.

The dry state of the fabric referred to in the present invention is achieved by leaving a test piece to stand for 24 hours or longer under a normal condition of 20 ± 2°C and 65 ± 2% RH atmosphere before measurement. Further, the wet state of the fabric refers to a condition of 30 seconds after adding 500 parts by mass of water to 100 parts by mass of the fabric in the dry state and then the added water having spread to the entirety of the fabric. The value obtained by dividing the static friction coefficient of the fabric in the wet state by the static friction coefficient of the fabric in the dry state is preferably 1.0 or more.

In the fabric of the present invention, the lower value of the tensile strengths between a longitudinal direction and a transverse direction in the dry state is 50 N/50 mm or more, and the lower value of the tensile strengths between the longitudinal direction and the transverse direction in the wet state is preferably 50 N/50 mm or more, and both of these values are more preferably 100 N/mm or more. By setting the lower value of the tensile strengths in the longitudinal direction and the transverse direction in the dry state to 50 N/50 mm or more, and the lower value of the tensile strengths in the longitudinal direction and the transverse direction in the wet state to 50 N/50 mm or more, it is possible to further improve the process passability. Hereinafter, when the fabric is a knitted fabric, the longitudinal direction is a direction parallel to a roller flow direction at the time of winding in the manufacturing process, and the transverse direction of the knitted fabric is a direction perpendicular to the longitudinal direction of the above knitted fabric. Also, when the fabric is a woven fabric, the longitudinal direction corresponds to the warp direction and the transverse direction corresponds to the weft direction.

The tensile stress at 30% elongation of the fabric of the present invention in the dry state is preferably 100 N/50 mm or less. By setting the tensile stress at 30% elongation of the fabric in the dry state to 100 N/50 mm or less, a fabric excellent in stretchability can be obtained. From the above viewpoint, the tensile stress is more preferably 80 N/50 mm or less, and even more preferably 50 N/50 mm or less. Further, the tensile stress at 30% elongation of the fabric of the present invention in the wet state is preferably 100 N/50 mm or less. By setting the tensile stress at 30% elongation of the fabric in the wet state to 100 N/50 mm or less, a fabric excellent in stretchability can be obtained. From the above viewpoint, the tensile stress is more preferably 80 N/50 mm or less, and even more preferably 50 N/50 mm or less.

In addition, the water retention rate of the fabric of the present invention is preferably 500% or less. By setting the water retention rate of the fabric to 500% or less, it is possible to suppress deterioration in workability using the fabric having heavier weight in a wet state. From the above viewpoint, the water retention rate is more preferably 450% or less, and further preferably 400% or less.

The fabric of the present invention is suitable for an application in which a feeling of use similar to in a dry state is required also in a wet state. Specific examples of this include surgical gloves used at medical sites. The current medical latex gloves become very slippery when being wet with blood, medicinal solution or the like during surgery, and are difficult to use in the case of handling organs and the like. If part of or whole of the outer surface of a latex glove is covered by the fabric of the present invention, the above problem can be solved. In addition, the same effect as described above can be obtained, for various gloves, such as golf gloves, baseball gloves, and outdoor gloves, that are expected to be used in rainy weather and with perspiration, and also for car seats, interior skin materials for chairs and sofas, nursing care sheets, supporters, and the like, by covering the part of or the whole of the outer surface thereof with the fabric of the present invention. Furthermore, since the static friction coefficient of the fabric of the present invention has an appropriate difference between the first surface and the second surface, the fabric of the present invention can be suitably used for a glove or the like desired to have the characteristics described above.

EXAMPLES

Hereinafter, the present invention will be described in detail based on examples, but the present invention is not limited thereto. The performance was measured by the following method in the examples.

[Method of Measurement]

(1) Melt Viscosity of Polymer

The melt viscosity of a polymer chip was measured by changing the strain rate in stages with CAPILOGRAPH 1B manufactured by Toyo Seiki Seisaku-sho after setting the water content of the polymer chip to 200 ppm or less by using a vacuum dryer. In this method of measurement, a capillary having a diameter of 1.0 mm and a length of 10 mm was used. In addition, the load of a load cell was set to 1 t, the measurement temperature was set to the same temperature as the spinning temperature in the spinning using the polymer to be measured, the shear rate was set to 1216 s$^{-1}$, and thus the melt viscosity was measured. The time from the start of introduction of a polymer sample into a heating furnace to the start of the measurement was set to 5 minutes, and the measurement was performed in a nitrogen atmosphere.

(2) Single Fiber Diameter of Filament Yarn

Metal was deposited on the surface of the fabric by using a metal vapor deposition apparatus (product name: JEC-3000 FC Auto Fine Coater) manufactured by JEOL, the sample was mounted on a super high resolution field emission electron scanning microscope (product name: SU 8010) manufactured by Hitachi, images of five parts were captured. The single fiber diameter was measured from 50 filament yarns randomly selected from these images and the average value thereof was determined.

(3) Mass of Fabric

The mass of the fabric per unit area (1m$^2$) was determined by a method prescribed in JIS L 1096 (2010) (8.4.2).

(4) Thickness of Fabric

The thickness of the fabric was determined by using a thickness measurement device (manufactured by TECLOCK) according to a method specified in JIS L 1096 (2010) (8.5.1) .

(5) Exposed Area of Filament Yarn A on One Surface of Fabric and Exposed Area of All Fibers

An image of one surface of the fabric was photographed by using an optical electron microscope (VHX-2000, manufactured by KEYENCE CORPORATION) and by setting the magnification to 100 times (low magnification lens VH-25) and the brightness to the maximum setting of 256 steps with a slider of an illumination lamp in camera settings. Here, photographing was performed by randomly selecting a photographing site from one side of the fabric. Here, the photographing was performed such that at least a part of an end portion of the fabric was not included in the photographed image. Next, by subjecting the obtained photographed image to image processing analysis, the exposed area (hereinafter referred to as total area I) of all the fibers on one face of the fabric and the exposed area of the fibers other than the filament yarn A on the one surface of the fabric (hereinafter referred to as total area II) were calculated, and the exposed area (hereinafter referred to as total area III) of the filament yarn A on the one surface of the fabric was obtained by subtracting the total area II from the total area I. Here, the total area II is calculated by using automatic area measurement mounted on an optical microscope (VHX-2000, manufactured by KEYENCE CORPORATION), setting a extraction method to "luminance", an extraction parameter to "bright", a threshold value to "25", and noise removal to "weak", and calculating the sum of areas detected under the above settings. Next, the total area II is calculated by using automatic area measurement mounted on an optical microscope (VHX-2000, manufactured by KEYENCE CORPORATION), setting a extraction method to "luminance", an extraction parameter to "bright", a threshold value to "73", and noise removal to "weak", and calculating the sum of areas detected under the above settings.

For example, the value obtained by dividing the exposed area of the filament yarn A on the first surface of the fabric by the exposed area, on the first surface of the fabric, of all fibers exposed on the first surface of the fabric is obtained by dividing the total area III on the first surface of the fabric by the total area I on the first surface of the fabric.

(6) Static Friction Coefficient

Based on a method prescribed in JIS P 8147 8 inclination method (2010), the static friction coefficient of the fabric was determined by setting a silicon plate (bio skin plate #2 manufactured by BULLRECS Co., Ltd.) as the contacted object. Specifically, the static friction coefficient was measured by using a slide inclination angle measurement device including an inclined plate. The inclined angle of the inclined plate was adjusted to zero, the silicon plate serving as the contacted object was fixed, a flat indenter stuck on the fabric was placed such that the silicon plate was in contact with the fabric, the inclination angle of the inclined plate was increased, and an inclination angle $\theta$ when the flat indenter began to slide together with the fabric was measured. From the obtained inclination angle $\theta$, the static friction coefficient $\mu$ was calculated by the following formula.

$$\mu = \tan\theta$$

The load was adjusted to 29 g/cm$^2$. Here, the load of 29 g/cm$^2$ is a pressure determined in consideration of evaluation of a grip property of gripping the contacted object.) In addition, depending on the stretchability of the fabric, the flat indenter may sometimes slide due to elongation of the fabric in the case where the frictional force between the fabric and the flat indenter is smaller than the frictional force between the fabric and the silicon plate. At this point, the sliding was not judged as the start of sliding, and the inclination angle when the flat indenter and the fabric began to slide together was measured as described above. The static friction coefficient of the fabric was measured five times for one direction of a randomly specified test piece, and the average value thereof was obtained as the static friction coefficient of the fabric. The dry state of the fabric referred to in the present invention is achieved by leaving a test piece to stand for 24 hours or longer under a normal condition of 20 ± 2°C and 65 ± 2% RH atmosphere before measurement. In addition, the wet state of the fabric is achieved by impregnating the fabric in a dry state in a water tank in which water of a sufficient amount to impregnate the entire fabric has been reserved and by pulling up the fabric from the water tank 30 seconds after the start of impregnation.

(7) Water Retention Rate of Fabric

Measurement was performed in accordance with a method according to JIS L 1906 (2000). Three test pieces of fabric of 10 cm × 10 cm were taken from the fabric and the mass of the test pieces of the fabric in the normal condition was measured to the unit of milligrams. The test piece was immersed in tap water at room temperature for 15 minutes, was removed from the water with tweezers, and after water was caused to drop for 1 minute, the mass thereof was measured to the unit of milligrams . The water retention rate was calculated by the following formula, and then the average value thereof was calculated.

$$m = (m_2 - m_1) / m_1 \times 100$$

Hereinafter, m: water retention rate (%)
$m_1$ : mass (mg) in the normal condition of the test piece
$m_2$ : mass (mg) after moistening the test piece and water was caused to drop.

The normal condition refers to a condition after the test piece of the fabric is left standing for 24 hours or longer in a 20 ± 2°C and 65 ± 2% RH atmosphere.

(8) Pilling Property

The pilling property of the fabric was measured by a method prescribed in JIS L 1076 (2010).

(9) Process Passability

Evaluation was made according to the following criteria for process passability of fabric in alkali weight reduction processing using a jet dyeing machine and finishing setting processing using a pin tenter machine.

A: None of clogging of a dyeing machine tube caused by adsorption of the fabric into the dyeing machine tube, tearing of the fabric caused by winding of the fabric on a guide roller of the pin tenter machine, meandering of the fabric, or detachment from a pin occurs.
B: Any one or more of clogging of a dyeing machine tube caused by adsorption of the fabric into the dyeing machine tube, tearing of the fabric caused by winding of the fabric on a guide roller of the pin tenter machine, meandering of the fabric, and detachment from a pin occurs.

(10) Tensile Strength of Fabric

The tensile strengths (N/50 mm) of the fabric in the longitudinal direction and the transverse direction in the dry state and the tensile strengths (N/50 mm) of the fabric in the longitudinal direction and the transverse direction in the wet state were measured by a method prescribed in JIS L 1096 8.14 (2010). A fabric having a length of 30 cm × a width of 5 cm (the longitudinal direction and the transverse direction) was attached to a tensile tester with a grip interval of 100 mm, a load was applied until the fabric was cut at a speed of 150 mm/min, and the strength of the maximum load was measured as the tensile strength in the dry state. Here, the dry state is achieved by leaving a test piece to stand for 24 hours or longer under a normal condition of 20 ± 2°C and 65 ± 2% RH atmosphere before measurement. Further, the fabric having a length of 30 cm × a width of 5 cm (the longitudinal direction and the transverse direction) in the dry state was immersed in water at 20°C for one hour, and was then taken out and attached to the tensile tester, and measurement of the tensile strength in the wet state was performed in the same manner as in the dry state. Measurement was performed three times, and the average value thereof was obtained.

(11) Tensile Stress at 30% Elongation When Fabric Is Wet

A test piece of the fabric having a length of 30 cm × a width of 5 cm was immersed in water at 20°C for one hour, and was then taken out and attached to the tensile tester with a grip interval of 100 mm, and the value of tensile stress (N/50 mm) at the time of 30% elongation at a speed of 150 mm/min was determined. Measurement was performed three times, and the average value thereof was obtained.

(12) Glove Removal Test

Using the obtained fabric, a glove was obtained such that the first surface thereof was on the outside (object gripping side) and the second surface was on the inside (on the hand side of the wearer of the glove), and evaluation was performed according to the following criteria with respect to a test for taking off the glove at the time of use.

A: The glove is easy to wear and take off in a dry state and a wet state.
B: The glove is difficult to wear and take off in a dry state or a wet state.

(13) Glove Grip Test (I)

Using the obtained fabric, a glove was obtained such that the first surface thereof was on the outside (object gripping side) and the second surface was on the inside (on the hand side of the wearer of the glove), and evaluation was performed according to the following criteria with respect to a grip test of the glove at the time of use.

A: Excellent in grip in a wet state.
B: Poor in grip in a wet state.

(14) Glove Grip Test (II)

Using the obtained fabric, a glove was obtained such that the first surface thereof was on the outside (object gripping side) and the second surface was on the inside (on the hand side of the wearer of the glove), and evaluation was performed on a grip test assuming use in surgery. Specifically, the grip of the glove was evaluated for ten subjects according to the following criteria by performing a test of evaluating the grip by wearing a glove obtained from the fabric of the present invention on a latex glove and grasping a commercially available konnyaku whose the surface was moistened by a solution prepared to contain 33% of glycerin (solution containing lipid). Then, comprehensive evaluation was made based on the total score of each person's evaluation.

<Individual Evaluation Criteria>

3 points : Excellent grip, easy to get the sense of the fingertip, and easy to work with.
2 points: Good grip, easy to get the sense of the fingertip, and easy to work with.
1 point: Poor grip, difficult to get the sense of the fingertip, and difficult to work with.

<Total Evaluation Criteria>

A (good): 25 to 30 points
B (acceptable): 17 to 25 points
C (poor): 10 to 16 points.

(15) Surface Quality of Glove

Using the obtained fabric, a glove was obtained such that the first surface thereof was on the outside (object gripping side) and the second surface was on the inside (on the hand side of the wearer of the glove), the outer surface of the glove was observed, and evaluation was performed according to the following criteria.

A: Neither wrinkles nor glazing are observed on the outer surface of the glove, and the surface quality of the glove is prominently excellent.
B: Only either of wrinkles or glazing is observed on the outer surface of the glove, and the surface quality of the glove is excellent.
C: Both of wrinkles and glazing are observed on the outer surface of the glove, and the surface quality of the glove is poor.

(Example 1)

An island component of nylon 6 (melt viscosity: 190 Pa·sec) and a sea component of PET (melt viscosity: 95 Pa·s) copolymerized with 8.0 mol% of sodium 5-sulfoisophthalate were injected into a spinning pack incorporating a composite spinneret, and composite polymer streams were ejected through ejection holes. The spinneret was configured as a spinneret for 30,000 islands in total by using an ejection plate having 15 ejection holes and a distribution plate having 2000 distribution holes for the island component per ejection hole as a distribution plate right above the ejection plate. The mass ratio of sea component/island component was set to 30/70, winding was performed at a spinning temperature of 260°C and a spinning speed of 1500 m/min, and thus undrawn fiber of 150 dtex-15 filaments (total ejection amount of 40 g/min) was collected. The wound-up undrawn fiber was stretched 3.0 times between rollers heated to 70°C and 130°C. The obtained sea-island composite fiber was 89 dtex-15 filaments, and had a strength of 2.9 cN/dtex and an elongation of 31%. In addition, as the filament yarn B, a multifilament having a total fineness of 78 dtex-24 filaments of nylon 6, a tensile strength of 4.1 cN/dtex, and an elongation of 42% was used.

The obtained sea-island composite fiber and filament yarn B were interlaced with each other at an interlace ratio of 52 : 48, and a circular knit fabric was obtained by knitting the sea-island composite fiber and filament yarn B by using a double circular knitting machine with a gauge of 28 and a pot diameter of 838.2 mm (33 inches) such that the knit structure was double pique, that a value obtained by dividing the exposed area of the filament yarn A on the first surface of the fabric after the alkali weight reduction process by the exposed area, on the first surface, of all fibers exposed on the first surface was 0.8, and that a value obtained by dividing the exposed area of the filament yarn A on the second surface of the fabric by the exposed area, on the second surface, of all the fibers on the second surface was 0.2.

This circular knitted fabric was subjected to alkali weight reduction processing using a jet dyeing machine. Specifically, the process is as follows.
The circular knitted fabric was placed in the jet dyeing machine with the first surface of the circular knitted fabric as the inside of a tubular knitted fabric and the second surface thereof in contact with the inside of a tube of the dyeing machine. The circular knitted fabric was treated with a 1% aqueous solution of sodium hydroxide at 95°C for 30 minutes to remove the sea component, and 99.9% or more of the polylactic acid in the sea-island composite fiber was removed by hydrolysis.

Thereafter, the circular knitted fabric was dried with a drier at 100°C for 2 minutes. After drying, the circular knitted fabric was cut open from the tubular knitted fabric, and the circular knitted fabric was dried in a drier at 130°C for 1 minute for finishing setting such that the second surface of the circular knitted fabric was in contact with a feeding guide roller, and a circular knitted fabric constituted by nanofibers of nylon 6 (filament yarn A) and multifilament of nylon 6 (filament yarn B) was obtained. Both the first surface and the second surface of the obtained circular knitted fabric had 30 courses/25.4 mm and 25 wales/25.4 mm.

The obtained fabric was evaluated. The results are shown in Table 1. The filament yarn A had a single fiber diameter of 480 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.78, and the value (second value) obtained by dividing the exposed area of the filament yarn A on the second surface of the fabric by the exposed area, on the second surface, of all fibers exposed on the second surface was 0.22. The process passability was A. In addition, the result of the glove removal test was A, the result of the glove grip test (I) was A, and the result of the glove grip test (II) was B. The surface quality of the glove was A.

(Example 2)

A fabric was obtained in the same manner as in Example 1 except that the mass ratio of the sea/island component was changed to 50/50 and the total ejection amount was changed to 20 g/min.

The obtained fabric was evaluated. The results are shown in Table 1. The filament yarn A had a single fiber diameter of 290 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.78, and the value (second value) obtained by dividing the exposed area of the filament yarn A on the second surface of the fabric by the exposed area, on the second surface, of all fibers exposed on the second surface was 0.22. The process passability was A. In addition, the result of the glove removal test was A, the result of the glove grip test (I) was A, and the result of the glove grip test (II) was A. The surface quality of the glove was A.

(Example 3)

A fabric was obtained in the same manner as in Example 1 except that the mass ratio of the sea/island component was changed to 70/30 and the total ejection amount was changed to 10 g/min.

The obtained fabric was evaluated. The results are shown in Table 1. The filament yarn A had a single fiber diameter of 150 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.78, and the value (second value) obtained by dividing the exposed area of the filament yarn A on the second surface of the fabric by the exposed area, on the second surface, of all fibers exposed on the second surface was 0.22. The process passability was A. In addition, the result of the glove removal test was A, the result of the glove grip test (I) was A, and the result of the glove grip test (II) was A. The surface quality of the glove was A.

(Example 4)

A fabric was obtained in the same manner as in Example 1 except that the total number of islands was changed to 7500/spinneret by changing the distribution plate right above the ejection plate to a distribution plate having 500 distribution holes for the island component per ejection hole, the mass ratio of the sea/island component was changed to 20/80, and the total ejection amount was changed to 35 g/min.

The obtained fabric was evaluated. The results are shown in Table 1. The filament yarn A had a single fiber diameter of 960 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.78, and the value (second value) obtained by dividing the exposed area of the filament yarn A on the second surface of the fabric by the exposed area, on the second surface, of all fibers exposed on the second surface was 0.22. The process passability was A. In addition, the result of the glove removal test was A, the result of the glove grip test (I) was A, and the result of the glove grip test (II) assuming use in surgery was B. The surface quality of the glove was A.

(Example 5)

A fabric was obtained in the same manner as in Example 1 except that the island component was changed to polyethylene terephthalate (melt viscosity: 160 Pa·sec).

The obtained fabric was evaluated. The results are shown in Table 1. The filament yarn A had a single fiber diameter of 480 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.78, and the value (second value) obtained by dividing the exposed area of the filament yarn A on the second surface of the fabric by the exposed area, on the second surface, of all fibers exposed on the second surface was 0.22. The process passability was A. In addition, the result of the glove removal test was A, the result of the glove grip test (I) was A, and the result of the glove grip test (II) was B. The surface quality of the glove was A.

(Example 6)

A fabric was obtained in the same manner as in Example 1 except that the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 0.5, and such that the value obtained by dividing the exposed area of the filament yarn A on the second surface of the fabric by the exposed area, on the second surface, of all fibers exposed on the second surface was 0.2.

The obtained fabric was evaluated. The results are shown in Table 2. The filament yarn A had a single fiber diameter of 480 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.52, and the value (second value) obtained by dividing the exposed area of the filament yarn A on the second surface of the fabric by the exposed area, on the second surface, of all fibers exposed on the second surface was 0.21. The process passability was A. In addition, the result of the glove removal test was A, the result of the glove grip test (I) was A, and the result of the glove grip test (II) was B. The surface quality of the glove was A.

(Example 7)

A fabric was obtained in the same manner as in Example 1 except that the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 0.9, and such that the value obtained by dividing the exposed area of the filament yarn A on the second surface of the fabric by the exposed area, on the second surface, of all fibers exposed on the second surface was 0.2.

The obtained fabric was evaluated. The results are shown in Table 2. The filament yarn A had a single fiber diameter of 480 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.90, and the value (second value) obtained by dividing the exposed area of the filament yarn A on the second surface of the fabric by the exposed area, on the second surface, of all fibers exposed on the second surface was 0.20. The process passability was A. In addition, the result of the glove removal test was A, the result of the glove grip test (I) was A, and the result of the glove grip test (II) was B. The surface quality of the glove was B.

(Example 8)

A fabric was obtained in the same manner as in Example 1 except that the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 0.8, and such that the value obtained by dividing the exposed area of the filament yarn A on the second surface of the fabric by the exposed area, on the second surface, of all fibers exposed on the second surface was 0.1.

The obtained fabric was evaluated. The results are shown in Table 2. The filament yarn A had a single fiber diameter of 480 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.80, and the value (second value) obtained by dividing the exposed area of the filament yarn A on the second surface of the fabric by the exposed area, on the second surface, of all fibers exposed on the second surface was 0.10. The process passability was A. In addition, the result of the glove removal test was A, the result of the glove grip test (I) was A, and the result of the glove grip test (II) was B. The surface quality of the glove was A.

(Example 9)

A fabric was obtained in the same manner as in Example 1 except that the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 0.8, and such that the value obtained by dividing the exposed area of the filament yarn A on the second surface of the fabric by the exposed area, on the second surface, of all fibers exposed on the second surface was 0.4.

The obtained fabric was evaluated. The results are shown in Table 2. The filament yarn A had a single fiber diameter of 480 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.80, and the value (second value) obtained by dividing the exposed area of the filament yarn A on the second surface of the fabric by the exposed area, on the second surface, of all fibers exposed on the second surface was 0.40. The process passability was "O". In addition, the result of the glove removal test was A, the result of the glove grip test (I) was A, and the result of the glove grip test (II) was B. The surface quality of the glove was A.

(Example 10)

A fabric was obtained in the same manner as in Example 1 except that nylon 6 multifilament having a total fineness of 33 dtex-26 filaments was used as the filament yarn B.

The obtained fabric was evaluated. The results are shown in Table 2. The filament yarn A had a single fiber diameter of 480 nm and the filament yarn B had a single fiber diameter of 12 $\mu$m. The value (first value) obtained by dividing the exposed area of the filament yarn A on the first surface of the fabric by the exposed area, on the first surface, of all fibers exposed on the first surface was 0.78, and the value (second value) obtained by dividing the exposed area of the filament yarn A on the second surface of the fabric by the exposed area, on the second surface, of all fibers exposed on the second surface was 0.22. The process passability was A. In addition, the result of the glove removal test was A, the result of the glove grip test (I) was A, and the result of the glove grip test (II) was B. The surface quality of the glove was A.

(Example 11)

A fabric was obtained in the same manner as in Example 2 except that the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 0.7, and such that the value obtained by dividing the exposed area of the filament yarn A on the second surface of the fabric by the exposed area, on the second surface, of all fibers exposed on the second surface was 0.3.

The obtained fabric was evaluated. The results are shown in Table 3. The filament yarn A had a single fiber diameter of 290 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.70, and the value (second value) obtained by dividing the exposed area of the filament yarn A on the second surface of the fabric by the exposed area, on the second surface, of all fibers exposed on the second surface was 0.30. The process passability was A. In addition, the result of the glove removal test was A, the result of the glove grip test (I) was A, and the result of the glove grip test (II) was A. The surface quality of the glove was A.

(Example 12)

A fabric was obtained in the same manner as in Example 2 except that the circular knitted fabric was obtained by, in the knitting, performing knitting such that the value (first value) obtained by dividing the exposed area of the filament yarn A on the first surface of the fabric after alkali reduction processing by the exposed area, on the first surface, of all fibers exposed on the first surface was 0.5, and such that the value (second value) obtained by dividing the exposed area of the filament yarn A on the second surface of the fabric by the exposed area, on the second surface, of all fibers exposed on the second surface was 0.5.

The obtained fabric was evaluated. The results are shown in Table 3. The filament yarn A had a single fiber diameter of 290 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.50, and the value (second value) obtained by dividing the exposed area of the filament yarn A on the second surface of the fabric by the exposed area, on the second surface, of all fibers exposed on the second surface was 0.50. The process passability was B. In addition, the result of the glove removal test was B, the result of the glove grip test (I) was A, and the result of the glove grip test (II) was A. The surface quality of the glove was A.

(Example 13)

A fabric was obtained in the same manner as in Example 2 except that the island component was changed to polyethylene terephthalate (melt viscosity: 160 Pa·sec).

The obtained fabric was evaluated. The results are shown in Table 3. The filament yarn A had a single fiber diameter of 290 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.80, and the value (second value) obtained by dividing the exposed area of the filament yarn A on the second surface of the fabric by the exposed area, on the second surface, of all fibers exposed on the second surface was 0.20. The process passability was A. In addition, the result of the glove removal test was A, the result of the glove grip test (I) was A, and the result of the glove grip test (II) was B. The surface quality of the glove was A.

(Example 14)

A fabric was obtained in the same manner as in Example 2 except that knitting was performed by making the filament yarn A and the filament yarn B each into a tripled yarn.
The obtained fabric was evaluated. The results are shown in Table 3. The filament yarn A had a single fiber diameter of 290 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.80, and the value (second value) obtained by dividing the exposed area of the filament yarn A on the second surface of the fabric by the exposed area, on the second surface, of all fibers exposed on the second surface was 0.20. The process passability was A. In addition, the result of the glove removal test was A, the result of the glove grip test (I) was B, and the result of the glove grip test (II) was C. The surface quality of the glove was A.

(Comparative Example 1)

A fabric was obtained in the same manner as in Example 1 except that polyethylene terephthalate (melt viscosity: 160 Pa·sec) was used as the island component, the total number of islands was set to 15000/spinneret by using a distribution plate having 1000 distribution holes for the island component per ejection hole as the distribution plate right above the ejection plate, and the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 1.0, and such that the value obtained by dividing the exposed area of the filament yarn A on the second surface of the fabric by the exposed area, on the second surface, of all fibers exposed on the second surface was 1.0.
The obtained fabric was evaluated. The results are shown in Table 4. The filament yarn A had a single fiber diameter of 680 nm and the filament yarn B had a single fiber diameter of 31 $\mu$m. The value (first value) obtained by dividing the exposed area of the filament yarn A on the first surface of the fabric by the exposed area, on the first surface, of all fibers exposed on the first surface was 1.00, and the value (second value) obtained by dividing the exposed area of the filament yarn A on the second surface of the fabric by the exposed area, on the second surface, of all fibers exposed on the second surface was 1.00. To be noted, adsorption of the fabric inside the pot for alkali weight reduction occurred, and thus the process passability was B.
In addition, the result of the glove removal test was B, the result of the glove grip test (I) was A, and the result of the glove grip test (II) was B. The surface quality of the glove was C.

(Comparative Example 2)

A fabric was obtained in the same manner as in Example 1 except that the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 0.4, and such that the value obtained by dividing the exposed area of the filament yarn A on the second surface of the fabric by the exposed area, on the second surface, of all fibers exposed on the second surface was 0.2.
The obtained fabric was evaluated. The results are shown in Table 4. The filament yarn A had a single fiber diameter of 480 nm and the filament yarn B had a single fiber diameter of 19 $\mu$m. The value (first value) obtained by dividing the exposed area of the filament yarn A on the first surface of the fabric by the exposed area, on the first surface, of all fibers exposed on the first surface was 0.40, and the value (second value) obtained by dividing the exposed area of the filament yarn A on the second surface of the fabric by the exposed area, on the second surface, of all fibers exposed on the second surface was 0.20. The process passability was A.
In addition, the result of the glove removal test was A, the result of the glove grip test (I) was B, and the result of the glove grip test was C (II). The surface quality of the glove was A.

(Example 15)

A fabric was obtained in the same manner as in Example 1 except that the circular knitted fabric was obtained by, in the knitting, performing knitting such that the value obtained by dividing the exposed area of the filament yarn A on the first surface of the fabric after alkali reduction processing by the exposed area, on the first surface, of all fibers exposed on the first surface was 0.8, and such that the value obtained by dividing the exposed area of the filament yarn A on the second surface of the fabric by the exposed area, on the second surface, of all fibers exposed on the second surface was 0.5.

The obtained fabric was evaluated. The results are shown in Table 4. The filament yarn A had a single fiber diameter of 480 nm and the filament yarn B had a single fiber diameter of 19 μm. The value (first value) obtained by dividing the exposed area of the filament yarn A on the first surface of the fabric by the exposed area, on the first surface, of all fibers exposed on the first surface was 0.80, and the value (second value) obtained by dividing the exposed area of the filament yarn A on the second surface of the fabric by the exposed area, on the second surface, of all fibers exposed on the second surface was 0.50. To be noted, adsorption of the fabric inside the pot for alkali weight reduction occurred, and thus the process passability was B.

In addition, the result of the glove removal test was B, the result of the glove grip test (I) was A, and the result of the glove grip test (II) was B. The surface quality of the glove was A.

(Comparative Example 3)

A fabric was obtained in the same manner as in Example 1 except that a sea-island composite fiber containing polyethylene terephthalate (PET, melt viscosity: 160 Pa·sec) as the island component and modified polyester copolymerized with 5 mol% of sodium 5-sulfoisophthalate as the sea component, having a composite ratio of sea/island components of 20/80, 66 dtex-9 filaments in total, 70 fibers of the island components, and a single island yarn fineness of 0.08 dtex after elution of the sea component.

The obtained fabric was evaluated. The results are shown in Table 4. The filament yarn obtained from the sea-island composite fiber had a single fiber diameter of 2000 nm and the filament yarn B had a single fiber diameter of 19 μm. The value (first value) obtained by dividing the exposed area of the filament yarn A on the first surface of the fabric by the exposed area, on the first surface, of all fibers exposed on the first surface was 0, and the value (second value) obtained by dividing the exposed area of the filament yarn A on the second surface of the fabric by the exposed area, on the second surface, of all fibers exposed on the second surface was 0. The process passability was A.

In addition, the result of the glove removal test was A, the result of the glove grip test (I) was B, and the result of the glove grip test (II) was C. The surface quality of the glove was A.

(Comparative Example 4)

A fabric was obtained in the same manner as in Example 1 except that the total number of islands was changed to 7500/spinneret by changing the distribution plate right above the ejection plate to a distribution plate having 500 distribution holes for the island component per ejection hole, the composite ratio of the sea/island component was changed to 10/90, the total ejection amount was changed to 40 g/min, and the fabric was constituted only by the filament yarn A without using the filament yarn B.

The obtained fabric was evaluated. The results are shown in Table 4. The single fiber diameter of the filament yarn was 1000 nm, the value (first value) obtained by dividing the exposed area of the filament yarn A on the first surface of the fabric by the exposed area, on the first surface, of all fibers exposed on the first surface was 1.00, and the value (second value) obtained by dividing the exposed area of the filament yarn A on the second surface of the fabric by the exposed area, on the second surface, of all fibers exposed on the second surface was 1.00. To be noted, adsorption of the fabric inside the pot for alkali weight reduction occurred, and thus the process passability was B.

In addition, the result of the glove removal test was B because the glove was difficult to wear or take off although the glove was excellent in grip in the dry state and the wet state, the result of the glove grip test (I) was A, and the result of the glove grip test (II) was B. The surface quality of the glove was C.

[Table 1]

[Table 1]

|  |  | Example 1 | Example 2 | Example 3 | Example 4 | Example 5 |
|---|---|---|---|---|---|---|
| Filament yarn A | Polymer | Nylon 6 | Nylon 6 | Nylon 6 | Nylon 6 | PET |
|  | Single fiber diameter (nm) | 480 | 290 | 150 | 960 | 480 |

(continued)

| | | | Example 1 | Example 2 | Example 3 | Example 4 | Example 5 |
|---|---|---|---|---|---|---|---|
| Filament yarn B | Polymer | | Nylon 6 | Nylon 6 | Nylon 6 | Nylon 6 | Nylon 6 |
| | Single fiber diameter ($\mu$m) | | 19 | 19 | 19 | 19 | 19 |
| Fabric | Mass (g/m$^2$) | | 130 | 143 | 140 | 140 | 130 |
| | Thickness (mm) | | 0.74 | 0.70 | 0.70 | 0.70 | 0.70 |
| | Ratio of exposed area of filament yarn A | First value | 0.78 | 0.78 | 0.78 | 0.78 | 0.78 |
| | | Second value | 0.22 | 0.22 | 0.22 | 0.22 | 0.22 |
| | Static friction coefficient (first surface) | In a dry state | 1.02 | 1.15 | 1.30 | 0.88 | 1.04 |
| | | In a wet state | 0.97 | 1.17 | 1.37 | 0.70 | 0.94 |
| | | (Wet state/dry state) | 0.95 | 1.02 | 1.05 | 0.79 | 0.90 |
| | Static friction coefficient (second surface) | In a wet state | 0.65 | 0.68 | 0.73 | 0.57 | 0.65 |
| | (Static friction coefficient of first surface in a wet state) / (Static friction coefficient of second surface in a wet state) | | 1.49 | 1.73 | 1.87 | 1.22 | 1.45 |
| | Tensile strength in a dry state (N/50 mm) | | 181 | 175 | 171 | 178 | 170 |
| | Tensile strength in a wet state (N/50 mm) | | 160 | 155 | 153 | 165 | 150 |
| | Tensile stress (N/50 mm) at 30% elongation in a wet state | | 33 | 29 | 24 | 35 | 30 |
| | Water retention rate (%) | | 360 | 380 | 410 | 320 | 340 |
| | Pilling property (grade) | | 4 | 4 | 3 | 4 | 4 |
| | Process Passability | | A | A | A | A | A |
| | Glove Removal Test | | A | A | A | A | A |
| | Glove Grip Test (I) | | A | A | A | A | A |
| | Glove Grip Test (II) | | B | A | A | B | B |
| | Surface Quality of Glove | | A | A | A | A | A |

[Table 2]

| | | | Example 6 | Example 7 | Example 8 | Example 9 | Example 10 |
|---|---|---|---|---|---|---|---|
| Filament yarn A | Polymer | | Nylon 6 | Nylon 6 | Nylon 6 | Nylon 6 | Nylon 6 |
| | Single fiber diameter (nm) | | 480 | 480 | 480 | 480 | 480 |
| Filament yarn B | Polymer | | Nylon 6 | Nylon 6 | Nylon 6 | Nylon 6 | Nylon 6 |
| | Single fiber diameter ($\mu$m) | | 19 | 19 | 19 | 19 | 12 |
| | Mass (g/m$^2$) | | 145 | 143 | 140 | 140 | 135 |
| | Thickness (mm) | | 0.71 | 0.68 | 0.70 | 0.70 | 0.68 |

(continued)

| | | | | Example 6 | Example 7 | Example 8 | Example 9 | Example 10 |
|---|---|---|---|---|---|---|---|---|
| Fabric | Ratio of exposed area of filament yarn A | First value | | 0.52 | 0.90 | 0.80 | 0.80 | 0.78 |
| | | Second value | | 0.21 | 0.20 | 0.10 | 0.40 | 0.22 |
| | Static friction coefficient (first surface) | In a dry state | | 0.85 | 1.23 | 1.00 | 1.00 | 0.98 |
| | | In a wet state | | 0.75 | 1.23 | 0.95 | 0.95 | 0.97 |
| | | (Wet state/dry state) | | 0.88 | 1.00 | 0.95 | 0.95 | 0.96 |
| | Static friction coefficient (second surface) | In a wet state | | 0.65 | 0.65 | 0.60 | 0.72 | 0.63 |
| | (Static friction coefficient of first surface in a wet state)/(Static friction coefficient of second surface in a wet state) | | | 1.15 | 1.89 | 1.58 | 1.32 | 1.54 |
| | Tensile strength in a dry state (N/50 mm) | | | 178 | 168 | 165 | 185 | 174 |
| | Tensile strength in a wet state (N/50 mm) | | | 165 | 155 | 158 | 168 | 159 |
| | Tensile stress (N/50 mm) at 30% elongation in a wet state | | | 30 | 35 | 31 | 30 | 30 |
| | Water retention rate (%) | | | 360 | 360 | 360 | 360 | 350 |
| | Pilling property (grade) | | | 4 | 4 | 4 | 4 | 4 |
| | Process Passability | | | A | A | A | A | A |
| | Glove Removal Test | | | A | A | A | A | A |
| | Glove Grip Test (I) | | | A | A | A | A | A |
| | Glove Grip Test (II) | | | B | B | B | B | B |
| | Surface Quality of Glove | | | A | B | A | A | A |

[Table 3]

| | | | Example 11 | Example 12 | Example 13 | Example 14 |
|---|---|---|---|---|---|---|
| Filament yarn A | Polymer | | Nylon 6 | Nylon 6 | PET | Nylon 6 |
| | Single fiber diameter (nm) | | 290 | 290 | 290 | 290 |
| Filament yarn B | Polymer | | Nylon 6 | Nylon 6 | Nylon 6 | Nylon 6 |
| | Single fiber diameter ($\mu$m) | | 19 | 19 | 19 | 19 |
| | Mass (g/m$^2$) | | 140 | 80 | 140 | 380 |
| | Thickness (mm) | | 0.70 | 0.68 | 0.70 | 1.20 |
| | Ratio of exposed area of filament yarn A | First value | 0.70 | 0.50 | 0.80 | 0.80 |
| | | Second value | 0.30 | 0.50 | 0.20 | 0.20 |
| | Static friction coefficient (first surface) | In a dry state | 1.08 | 1.05 | 1.10 | 1.15 |
| | | In a wet state | 1.15 | 1.12 | 0.95 | 1.18 |
| | | (Wet state/dry state) | 1.06 | 1.07 | 0.86 | 1.03 |

(continued)

| | | | Example 11 | Example 12 | Example 13 | Example 14 |
|---|---|---|---|---|---|---|
| Fabric | Static friction coefficient (second surface) | In a wet state | 0.74 | 1.05 | 0.65 | 0.68 |
| | (Static friction coefficient of first surface in a wet state)/(Static friction coefficient of second surface in a wet state) | | 1.55 | 1.07 | 1.46 | 1.74 |
| | Tensile strength in a dry state (N/50 mm) | | 170 | 190 | 170 | 220 |
| | Tensile strength in a wet state (N/50 mm) | | 150 | 165 | 155 | 195 |
| | Tensile stress (N/50 mm) at 30% elongation in a wet state | | 28 | 30 | 28 | 38 |
| | Water retention rate (%) | | 370 | 380 | 340 | 370 |
| | Pilling property (grade) | | 4 | 4 | 4 | 4 |
| | Process Passability | | A | B | A | A |
| | Glove Removal Test | | A | B | A | A |
| | Glove Grip Test (I) | | A | A | A | B |
| | Glove Grip Test (II) | | A | A | B | C |
| | Surface Quality of Glove | | A | A | A | A |

[Table 4]

| | | | Comparative Example 1 | Comparative Example 2 | Example 15 | Comparative Example 3 | Comparative Example 4 |
|---|---|---|---|---|---|---|---|
| Filament yarn A | Polymer | | PET | Nylon 6 | Nylon 6 | - | Nylon 6 |
| | Single fiber diameter (nm) | | 680 | 480 | 480 | - | 1000 |
| Filament yarn B | Polymer | | Nylon 6 | Nylon 6 | Nylon 6 | Nylon 6 | - |
| | Single fiber diameter ($\mu$m) | | 31 | 19 | 19 | 19 | - |
| Fabric | Mass (g/m²) | | 140 | 138 | 140 | I 140 | 140 |
| | Thickness (mm) | | 0.70 | 0.70 | 0.70 | 0.70 | 0.68 |
| | Ratio of exposed area of filament yarn A | First value | 1.00 | 0.40 | 0.80 | 0 | 1.00 |
| | | Second value | 1.00 | 0.20 | 0.50 | 0 | 1.00 |
| | Static friction coefficient (first surface) | In a dry state | 0.95 | 0.74 | 1.00 | 0.78 | 0.97 |
| | | In a wet state | 0.80 | 0.67 | 0.95 | 0.57 | 0.81 |
| | | (Wet state/dry state) | 0.84 | 0.90 | 0.95 | 0.73 | 0.84 |
| | Static friction coefficient (second surface) | In a wet state | 0.80 | 0.64 | 0.84 | 0.53 | 0.81 |
| | (Static friction coefficient of first surface in a wet state) /(Static friction coefficient of second surface in a wet state) | | 1.00 | 1.05 | 1.13 | 1.07 | 1.00 |
| | Tensile strength in a dry state (N/50 mm) | | 170 | 175 | 186 | 180 | 173 |
| | Tensile strength in a wet state (N/50 mm) | | 151 | 158 | 168 | 155 | 150 |
| | Tensile stress (N/50 mm) at 30% elongation in a wet state | | 40 | 30 | 27 | 36 | 40 |
| | Water retention rate (%) | | 440 | 340 | 360 | 300 | 310 |
| | Pilling property (grade) | | 4 | 4 | 4 | 4 | 4 |
| | Process Passability | | B | A | B | A | B |
| | Glove Removal Test | | B | A | B | A | B |
| | Glove Grip Test (I) | | A | B | A | B | A |
| | Glove Grip Test (II) | | B | C | B | C | B |

(continued)

| | Comparative Example 1 | Comparative Example 2 | Example 15 | Comparative Example 3 | Comparative Example 4 |
|---|---|---|---|---|---|
| Surface Quality of Glove | C | A | A | A | C |

**Claims**

1. A fabric comprising a filament yarn A having a single fiber diameter of 100 to 1000 nm and a filament yarn B having a single fiber diameter of 10 $\mu$m or more, wherein a value obtained by dividing an exposed area of the filament yarn A on a first surface of the fabric by an exposed area, on the first surface, of all fibers exposed on the first surface is 0.50 or more and 0.90 or less.

2. The fabric according to claim 1, wherein a value obtained by dividing an exposed area of the filament yarn A on a second surface of the fabric by an exposed area, on the second surface, of all fibers exposed on the second surface is 0.10 or more and 0.40 or less.

3. The fabric according to claim 2, wherein a value obtained by dividing a static friction coefficient of the first surface in a wet state by a static friction coefficient of the second surface in a wet state is 1.2 or more and 2.5 or less, and the static friction coefficient of the first surface in the wet state is 0.7 or more.

4. The fabric according to any one of claims 1 to 3, wherein a tensile stress at 30% elongation in a wet state is 100 N/50 mm or less.

5. The fabric according to any one of claims 1 to 4, wherein the filament yarn A is a polyamide fiber and has a single fiber diameter of 100 to 300 nm.

6. A glove comprising the fabric according to any one of claims 1 to 5, wherein the fabric has a thickness of 0.2 to 0.9 mm.

7. The glove according to claim 6 used for surgery.

**INTERNATIONAL SEARCH REPORT**

International application No.

PCT/JP2016/065564

A. CLASSIFICATION OF SUBJECT MATTER
*D04B1/16*(2006.01)i, *D03D15/00*(2006.01)i, *D04B1/28*(2006.01)i, *A41D19/00*
(2006.01)n, *A41D19/015*(2006.01)n

According to International Patent Classification (IPC) or to both national classification and IPC

B. FIELDS SEARCHED

Minimum documentation searched (classification system followed by classification symbols)
A41D19/00-19/04, D03D1/00-27/18, D04B1/00-39/08

Documentation searched other than minimum documentation to the extent that such documents are included in the fields searched
Jitsuyo Shinan Koho        1922-1996   Jitsuyo Shinan Toroku Koho   1996-2016
Kokai Jitsuyo Shinan Koho  1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2014-210986 A  (Teijin Ltd.),<br>13 November 2014 (13.11.2014),<br>paragraph [0034]; examples<br>(Family: none) | 1,2,4,6,7<br>3,5 |
| X<br>A | JP 2010-024570 A  (Teijin Fibers Ltd.),<br>04 February 2010 (04.02.2010),<br>examples<br>(Family: none) | 1,2,4<br>3,5-7 |
| A | JP 2007-169829 A  (Toray Industries, Inc.),<br>05 July 2007 (05.07.2007),<br>(Family: none) | 1-7 |
| A | JP 2010-216036 A  (Teijin Fibers Ltd.),<br>30 September 2010 (30.09.2010),<br>(Family: none) | 1-7 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
09 August 2016 (09.08.16)

Date of mailing of the international search report
16 August 2016 (16.08.16)

Name and mailing address of the ISA/
Japan Patent Office
3-4-3,Kasumigaseki,Chiyoda-ku,
Tokyo 100-8915,Japan

Authorized officer

Telephone No.

Form PCT/ISA/210 (second sheet) (January 2015)

**INTERNATIONAL SEARCH REPORT**

International application No.

PCT/JP2016/065564

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2009-024272 A (Teijin Fibers Ltd.), 05 February 2009 (05.02.2009), (Family: none) | 1-7 |
| A | JP 2010-007186 A (Teijin Fibers Ltd.), 14 January 2010 (14.01.2010), (Family: none) | 1-7 |
| A | JP 2011-117099 A (Teijin Fibers Ltd.), 16 June 2011 (16.06.2011), (Family: none) | 1-7 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009024278 A

- WO 2012173116 A